# EUROPÄISCHE PATENTANMELDUNG

(11) **EP 2 157 076 A1**
(43) Veröffentlichungstag der Anmeldung: **24.02.2010**
(21) Anmeldenummer: 08014838.0
(22) Anmeldetag: 21.08.2008
(51) Int. Cl.: C07C 67/475, C07C 69/593, C07C 6/04, C07C 11/02, C07C 51/353, C07C 57/13, C11C 3/00

(54) **Verfahren zur Herstellung von ungesättigten alpha, omega Dicarbonsäurediestern**

(71) Anmelder: Cognis IP Management GmbH, 49589 Düsseldorf (DE)
(72) Erfinder: Samorski, Markus, 40217 Dusseldorf (DE); Dierker, Markus, 40593 Dusseldorf (DE)

(57) **Zusammenfassung**

Verfahren zur Herstellung von ungesättigten alpha, omega Dicarbonsäurediestern, bei dem man ungesättigte Fettsäuren und/oder Ester ungesättigter Fettsäuren in Gegenwart des Katalysators (1) und/oder (2) 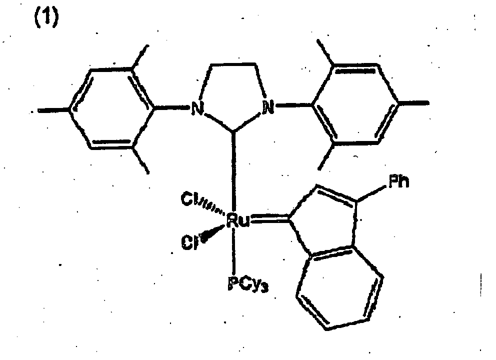 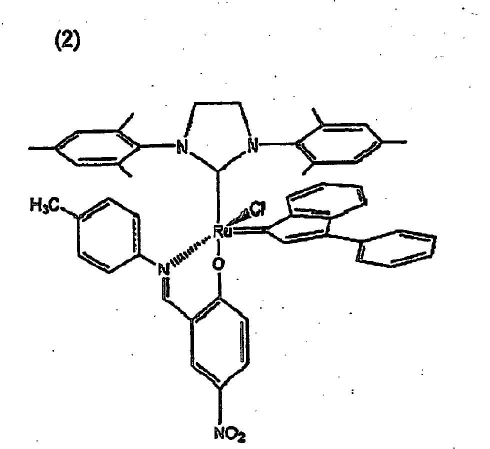 umzetzt.

## Beschreibung

Die vorliegende Erfindung betrifft ein Verfahren zur Herstellung von ungesättigten alpha,omega Dicarbonsäurediestern unter Einsatz zweier Ruthenium KomplexKatalysatoren.

Verfahren zur Herstellung von ungesättigten alpha,omega Dicarbonsäurediestern ausgehend von ungesättigten Fettsäuren sind im Stand der Technik beschrieben: Ngo et al. (JAOCS, Vol 83, No. 7, 2006**)** beschreibt die Metathese von ungesättigten Fettsäuren mit Hilfe der sogenannten Grubbs-Katalysatoren der ersten und zweiten Generation. Das Verfahren mit Grubbs-Katalysatoren der ersten Generation ist in US 5,750,815 beschrieben. Auch J. Mol (Topics in Catalysis, Vo. 27, Nos. 1-4, Feb. 2004) sowie A. Rybak et al (Green Chem. 2007, 9, 1356-1361**)** beschreiben solche Verfahren. Nachteilig an den Verfahren, welche die Grubbs-Katalysatoren der ersten Generation einsetzen, sind niedrige Ausbeuten und sehr langen Reaktionszeiten. Nachteilig an den Verfahren, welche Grubbs-Katalysatoren der zweiten Generation einsetzen, ist das Nebenproduktspektrum, welches eine aufwendige Reinigung der Endprodukte erfordert. Hierbei sind insbesondere Nebenprodukte störend, welche sich aus Edukten mit Katalysatorbestandteilen bilden.

Die Aufgabe der vorliegenden Erfindung bestand darin, ein neues Verfahren zu Verfügung zu stellen, welches er ermöglicht alpha, omega Dicarbonsäurediester in hoher Ausbeute, mit geringen Reaktionszeiten sowie in hoher Reinheit zu erhalten. Von Interesse war es weiterhin möglichst geringe Katalysatormengen einzusetzen. Überraschenderweise wurde gefunden, dass diese Aufgabe mit dem erfindungsgemäßen Verfahren gelöst wird.

Gegenstand der Erfindung ist ein Verfahren zur Herstellung von ungesättigten alpha, omega Dicarbonsäurediestern, bei dem man ungesättigte Fettsäuren und/oder Ester ungesättigter Fettsäuren in Gegenwart des Katalysators (1) und/oder (2) 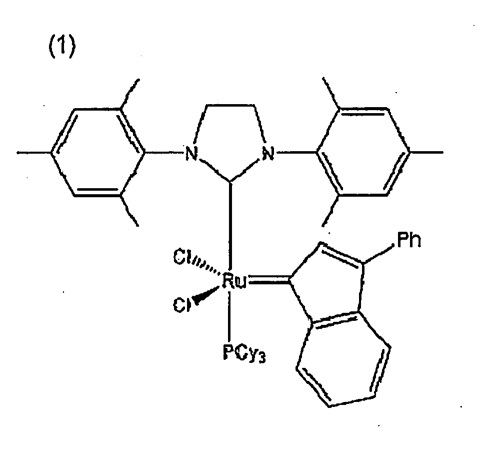 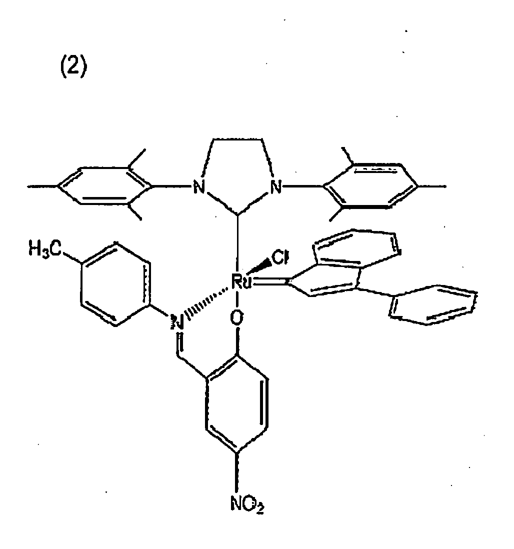 umsetzt.

### Edukte

Als Edukte für das erfindungsgemäße Verfahren eignen sich einfach oder mehrfach ungesättigte Fettsäuren mit 14 bis 24 Kohlenstoffatomen, die gegebenenfalls verzweigt sein können. Die Doppelbindungen der Fettsäuren können dabei sowohl in cis als auch in trans Konfiguration vorliegen.

Die folgende Kurzschreibweise wird zur Beschreibung der ungesättigten Fettsäuren verwendet: die erste Zahl beschreibt die Gesamtzahl der C Atome der Fettsäure, die zweite Zahl die Anzahl der Doppelbindungen, und die Zahl in Klammern die Position der Doppelbindung im Verhältnis zur Carbonsäuregruppe. Somit ist die Kurzschreibweise für Ölsäure 18:1 (9). Sofern die Doppelbindung in trans-Konfiguration vorliegt, ist dies durch das Kürzel "tr" gekennzeichnet. Somit ist die Kurzschreibweise für Elaidinsäure 18:1 (tr9).

Geeignete einfach ungesättigte Fettsäuren sind beispielsweise **Myristoleinsäuren** [14:1 (9), (9*Z*)- Tetradeca-9-ensäure], **Palmitoleinsäure** [16:1 (9); (9*Z*)- Hexadeca-9-ensäure], **Petroselinsäure** [(6*Z*)- Octadeca-6-ensäure], **Ölsäure** [18:1 (9); (9*Z*)-Octadeca-9-ensäure], **Elaidinsäure** [18:1 (tr9); (9*E*)-Octadeca-9-ensäure)], **Vaccensäure** [18:1 (tr11); (11*E*)- Octadeca-11-ensäure], **Gadoleinsäure** [20:1 (9); (9*Z*)- Eicosa-9-ensäure], **Icosensäure** (=Gondosäure) [20:1 (11); (11*Z*)- Eicosa- 11-ensäure], **Cetoleinsäuren** [22:1 (11); (11*Z*)- Docosa-11-ensäure], **Erucasäure** [22:1 (13); (13*Z*)- Docosa-13-ensäure], **Nervonsäure** [24:1 (15); (15*Z*)-Tetracosa-15-ensäure].

Geeignete mehrfach ungesättigte Fettsäuren sind beispielsweise **Linolsäure** [18:2 (9,12); (9*Z*,12*Z*)- Octadeca-9,12-diensäure], **alpha-Linolensäure** [18:3 (9,12,15); (9Z,12Z,15*Z*)- Octadeca-9,12,15-triensäure], **gamma-Linolensäure** [18:3 (6,9,12); (6*Z*,9*Z*,12*Z*)-Octadeca- 6,9,12-triensäure], **Calendulasäure** [18:3 (8,10,12); (8*E*,10*E*,12*Z*)-Octadeca-8,10,12- triensäure], **Punicinsäure** [18:3 (9,11,13); (9*Z*,11*E*,13*Z*)-Octadeca-9,11,13-triensäurej, **alpha-Eleostearinsäure** [18:3 (9,11,13); (9*Z*,11*E*,13*E*)-Octadeca-9,11,13-triensäure], **Arachidonsäure** [20:4 (5,8,11,14), (5*Z*,8*Z*,11*Z*,14*Z*)-Eicosa-5,8,11,14-tetraensäure], **Timnodonsäure** [20:5 (5,8,11,14,17), (5*Z*,8*Z*,11*Z*,14*Z*,17*Z*)-Eicosa-5,8,11,14,17-pentaensäure], **Clupandodonsäure** [22:5 (7,10,13,16,19), (7*Z*,10*Z*,13*Z*,16*Z*,19*Z*)-Docosa-7,10,13,16,19-pentaensäure], **Cervonsäure** [22:6 (4,7,10,13,16,19), (4*Z*,7*Z*,10*Z*,13*Z*,16*Z*, 19*Z*) -Docosa- 4,7,10,13,16,19-hexaensäure].

Als Edukte eignen sich weiterhin **Ester** der genannten einfach oder mehrfach ungesättigten Fettsäuren. Als Ester eignen sich insbesondere Ester dieser Fettsäuren mit Alkoholen R₁-OH, wobei R₁ ein Alkylrest mit 1 bis 8 C-Atomen darstellt. Als geeignete Reste R₁ seien beispielsweise genannt: Methyl-, Ethyl-, Propyl-, Isopropyl-, Butyl-, 2-Methylpropyl-, Pentyl-, 2,2-Dimethylpropyl-, 2-Methylbutyl-, 3-Methylbutyl-, Hexyl-, 1-Methylpentyl-, 2-Methylpentyl-, 3-Methylpentyl-, 4-Methylpentyl-, 1-Ethylbutyl-, 2-Ethylbutyl-, Heptyl-, und Octyl-Reste.

Als Edukte eignen sich weiterhin Ester der genannten einfach oder mehrfach ungesättigten Fettsäuren mit Glycerin (= Glycerinester). Dabei eignen sich sowohl Glycerinmonoester (= Monoglyceride, Monoacylglycerol), Glycerindiester (=Diglyceride, Diacylglycerol) als auch Glycerintriester (=Triglyceride, Triacylglycerol) als auch Mischungen dieser verschiedenen Glycerinester.

Die ungesättigten Fettsäuren bzw. die Ester der ungesättigten Fettsäuren können sowohl einzeln als auch in Mischungen untereinander vorliegen. Wird ausschließlich eine ungesättigte Fettsäure bzw. der Ester nur einer ungesättigten Fettsäure eingesetzt, so findet eine als Selbstmetathese bezeichnete Reaktion statt. Werden verschiedene ungesättigte Fettsäuren bzw. Ester verschiedener ungesättigte Fettsäuren eingesetzt, findet eine als Kreuz-Metathese bezeichnete Reaktion statt.

In einer bevorzugten Ausführungsform der Erfindung werden einfach ungesättigte Fettsäuren und/oder Ester einfach ungesättigter Fettsäuren und/oder Mischungen der einfach ungesättigten Fettsäuren oder Mischungen der Ester einfach ungesättigter Fettsäuren eingesetzt.

### Katalysator

Das erfindungsgemäße Verfahren wird in Gegenwart der Katalysatoren (1) und/oder (2) durchgeführt.

Katalysator (1) ist die Verbindung der folgenden Strukturformel:

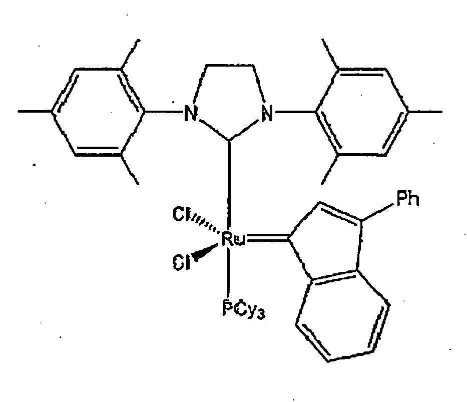

Die chemische Bezeichnung für diesen Katalysator ist Dichloro-[1,3-bis(mesityl)-2-imidazolidinylidene]-(3-phenyl-1H-inden-1-ylidene)(tricyclohexylphosphine)ruthenium(II), CAS Nr. 536724-67-1. Er ist kommerziell beispielsweise erhältlich unter der Bezeichnung Neolyst™M2 der Fa. Umicore.

Katalysator (2) ist die Verbindung der folgenden Strukturformel:

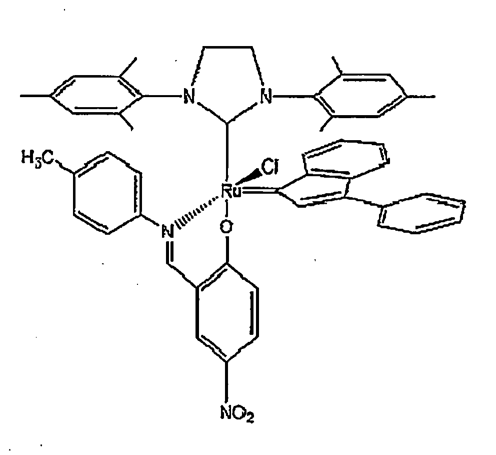

Die chemische Bezeichnung für diesen Katalysator ist [1,3-Bis(2,4,6-trimethylphenyl)-2-imidazolidinylidene]-[2-[[(4-methylphenyl)imino]methyl]-4-nitrophenolyl]-[3-phenyl-1H-inden-1-ylidene]ruthenium(II) chloride, CAS Nr. 934538-04-2. Er ist kommerziell beispielsweise erhältlich unter der Bezeichnung Neolyst™M41 der Fa. Umicore.

Der erfindungsgemäße Katalysator wird vorzugsweise in Konzentrationen kleiner gleich 1000 ppm, insbesondere im Bereich von 10 bis 1000 ppm, vorzugsweise 20 bis 200 ppm eingesetzt.

### Reaktionsbedingungen

Das erfindungsgemäße Verfahren kann bei Temperaturen von 0 bis 100°C, vorzugsweise bei 20 bis 90°C, insbesondere bei 40 bis 80°C durchgeführt werden.

Das Verfahren kann in üblichen Lösungsmitteln, in denen sich die Edukte sowie der Katalysator lösen, wie beispielsweise seien genannt: Kohlenwasserstoffe oder Alkohole. In einer bevorzugten Ausführungsform der Erfindung kann das Verfahren lösungsmittelfrei durchgeführt werden.

### Aufreinigung

Durch das erfindungsgemäße Verfahren werden alpha, omega Dicarbonsäurediester erhalten sowie die entsprechenden ungesättigten Kohlenwasserstoffe. Eine Trennung des Stoffgemisches kann z.B. destillativ, durch fraktionierte Kristallisation oder durch Extraktion erfolgen.

### Beispiele

### Beispiel 1a)

50 ml einer Methylester-Mischung (bestehend aus 92,0 % Methyloleat und 2,9 % Methyllinoleat, Prozentangaben beziehen sich auf eine nicht-kalibrierte Flächen-Prozent-GC-Methode) wurden 1,4 h bei 50°C in Gegenwart von 200 ppm des Katalysators (1) [CAS Nr. 536727-67-1] umgesetzt. Nach Beendigung der Reaktion wurden 23,1 % Dimethylesters sowie 20,2 % 9-Octadecen erhalten.

### Beispiel 1b)

Beispiel 1a) wurde wiederholt, allerdings wurde die Reaktion bei 40°C für 4 h durchgeführt. Die Ausbeute war identisch zu Beispiel 1 a).

### Beispiel 1c)

Beispiel 1a) wurde wiederholt, allerdings wurde die Reaktion bei 30°C für 7 h durchgeführt, Die Ausbeute war identisch zu Beispiel 1a).

### Beispiel 2)

50 ml einer Methylester-Mischung (bestehend aus 92,0 % Methyloleat und 2,9 % Methyllinoleat) wurden 8 h bei 80 °C in Gegenwart von 200 ppm des Katalysators (2) [CAS Nr. 934538-04-2] umgesetzt. Nach Beendigung der Reaktion wurden 23,3 % Dimethylester sowie 20,6 % 9-Octadecen erhalten.

## Patentansprüche

1. Verfahren zur Herstellung von ungesättigten alpha, omega Dicarbonsäurediestern, bei dem man ungesättigte Fettsäuren und/oder Ester ungesättigter Fettsäuren in Gegenwart des Katalysators (1) und/oder (2) 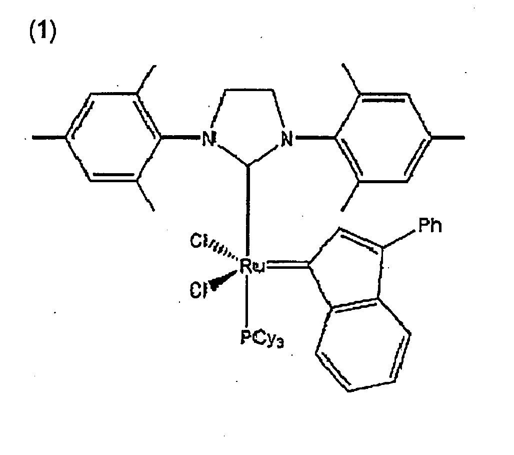 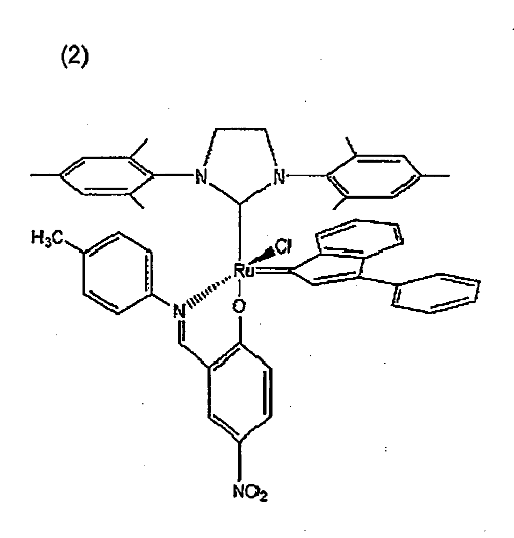 umsetzt.

2. Verfahren nach Anspruch 1, **dadurch gekennzeichnet, dass** man ungesättigte C14 bis C24 Fettsäuren einsetzt.

3. Verfahren nach Anspruch 1, **dadurch gekennzeichnet, dass** man Ester von ungesättigten Fettsäuren einsetzt, wobei die ungesättigte Fettsäure eine C14 bis C24 Fettsäure darstellt.

4. Verfahren nach Anspruch 3, **dadurch gekennzeichnet, dass** man als Ester einen Ester von C1 bis C8 Alkoholen mit ungesättigten Fettsäuren und/oder einen Ester von Glycerin mit ungesättigten Fettsäuren einsetzt.

5. Verfahren nach Anspruch 4, **dadurch gekennzeichnet, dass** der Glycerinester ausgewählt ist aus der Gruppe bestehend aus Glycerinmonoester, Glycerindiester und Glycerintriester.

6. Verfahren nach einem der vorgenannten Ansprüche, **dadurch gekennzeichnet, dass** man einfach ungesättigte Fettsäuren einsetzt.

7. Verfahren nach einem der vorgenannten Ansprüche, **dadurch gekennzeichnet, dass** man den Katalysator in einer Menge von 10 bis 1000 ppm, vorzugsweise von 20 bis 200 ppm einsetzt.

8. Verfahren nach einem der vorgenannten Ansprüche, **dadurch gekennzeichnet**, **dadurch gekennzeichnet, dass** man die Reaktion bei 0 bis 100°C, vorzugsweise bei 20 bis 90°C, insbesondere bei 40 bis 80°C durchführt.

9. Verfahren nach einem der vorgenannten Ansprüche, **dadurch gekennzeichnet, dass** man lösungsmittelfrei arbeitet.

10. Verfahren nach einem der vorgenannten Ansprüche, **dadurch gekennzeichnet, dass** man die alpha, omega Dicarbonsäurediester und ungesättigten Kohlenwasserstoffen mittels Destillation trennt.
